# EUROPEAN PATENT APPLICATION

(11) **EP 3 944 249 A1**
(43) Date of publication of application: **26.01.2022**
(21) Application number: 20773585.3
(22) Date of filing: 17.03.2020
(51) Int. Cl.: G16H 10/20, G16H 10/60, G16H 50/30, G01N 33/98

(54) **INTOXICATION DEGREE DETERMINATION SYSTEM, INTOXICATION DEGREE DETERMINATION METHOD, AND INTOXICATION DEGREE DETERMINATION PROGRAM**

(30) Priority: 18.03.2019 JP 2019050345
(71) Applicant: Suntory Holdings Limited, Osaka 530-8203 (JP)
(72) Inventor: SUZUKI Yuichi, Tokyo 135-8631 (JP); ZEIDA Mitsuhiro, Soraku-gun, Kyoto 619-0284 (JP); HASEGAWA Toshinobu, Osaka-shi, Osaka 530-8204 (JP); ENDO Koichi, Osaka-shi, Osaka 530-8204 (JP); KANEDA Sonoko, Osaka-shi, Osaka 530-8204 (JP); TAKAGI Motoshige, Osaka-shi, Osaka 530-8204 (JP); NAKAGAWA Aiko, Osaka-shi, Osaka 530-8204 (JP)
(74) Representative: Vossius & Partner Patentanwälte Rechtsanwälte mbB
(86) International application number: PCT/JP2020/011653
(87) International publication number: WO 2020/189667

(57) **Abstract**

The present disclosure includes a question creating section 21 configured to create a question for a drinking user to answer; an output section 11 configured to present the user with the question; an input section 11 configured to receive an operation by the user of inputting an answer to the question; and a computation section 22 configured to determine an intoxication degree of the user's on the basis of the answer.

## Description

### Technical Field

The present invention relates to an intoxication degree determining system, an intoxication degree determining method, and an intoxication degree determining program each of which allows a user's intoxication degree to be determined.

### Background Art

Development is currently underway of a system having a function of determining a user's intoxication degree and a function of causing a device to perform a predetermined operation on the basis of the intoxication degree determined, in order to prevent such drinking-induced events as an injury in an accident or a leak of information managed by a company.

Japanese Unexamined Patent Application Publication, Tokukai, No. 2017-183783 (Patent Literature 1), for example, discloses a technique of learning the path of a user's normal flick input operation on a smartphone and determining that the user is intoxicated if a flick input operation by the user has a path significantly different from the normal path learned as above.

Further, Japanese Unexamined Patent Application Publication, Tokukai, No. 2016-178455 (Patent Literature 2) discloses a technique of providing an information processing device with a biological information sensor such as a skin gas sensor or a breath sensor and determining a user's intoxication state on the basis of the result of detection by the sensor.

### Citation List

### Patent Literature

Patent Literature 1 Japanese Unexamined Patent Application Publication, Tokukai, No. 2017-183783
Patent Literature 2 Japanese Unexamined Patent Application Publication, Tokukai, No. 2016-178455

### Summary of Invention

### Technical Problem

The technique of Patent Literature 1 unfortunately requires learning the path of a user's normal input operation, and may not allow the user's intoxication state to be determined accurately if the learning has not been conducted yet or is insufficient. The technique of Patent Literature 2 may fail to make an accurate measurement as a result of the user's drinking environment (such as a restaurant) being in many cases not necessarily suitable for the measurement in terms of lightness, color, and atmosphere.

The above circumstances have led to a demand for an intoxication degree determining system, an intoxication degree determining method, and an intoxication degree determining program each capable of determining a user's intoxication state without requiring prior learning or accurate measurement.

### Solution to Problem

An intoxication degree determining system of the present invention includes: a question creating section configured to create a question for a drinking user to answer; an output section configured to present the user with the question; an input section configured to receive an operation by the user of inputting an answer to the question; and a computation section configured to determine an intoxication degree of the user's on a basis of the answer.

An intoxication degree determining method of the present invention includes: creating a question for a drinking user to answer; presenting the user with the question; receiving an answer to the question from the user; and determining an intoxication degree of the user's on a basis of the answer.

An intoxication degree determining program of the present invention is configured to cause a computer to: create a question for a drinking user to answer; present the user with the question; receive an input of an answer to the question from the user; and determine an intoxication degree of the user's on a basis of the answer.

The above arrangements each allow a user's intoxication state to be determined without requiring prior learning or accurate measurement.

The description below deals with preferable embodiments of the present invention. The preferable embodiments described below as examples do not limit the scope of the present invention.

An intoxication degree determining system as a preferable embodiment of the present invention further includes: a sensor section configured to sense at least one of a pulse rate, a blood pressure, and a blood alcohol concentration of the user's, wherein the computation section determines the intoxication degree of the user's additionally on a basis of the at least one of the pulse rate, the blood pressure, and the blood alcohol concentration of the user's.

The above arrangement allows the intoxication degree to be determined on the basis of both the answer to the question and the measurement result, and is thereby likely to allow the intoxication degree to be determined more accurately.

An intoxication degree determining system as a preferable embodiment of the present invention is arranged such that the computation section determines on a basis of the intoxication degree whether the user is able to continue drinking, and the output section is configured to present the use with a result of the determination of whether the user is able to continue drinking.

The above arrangement allows the user to be advised to drink moderately, and is thereby likely to increase the level of satisfaction on drinking occasions.

An intoxication degree determining system as a preferable embodiment of the present invention further includes: a history storing section configured to store a history of the intoxication degree of the user's, wherein the question creating section is configured to create a follow-up question, the follow-up question being a question for the user to answer about a health condition of the user's at a follow-up time point, the follow-up time point being a predetermined time point after the user ended drinking, the history storing section is configured to further store a history of the health condition, which is based on an answer by the user to the follow-up question, and the computation section is configured to determine a tendency of the user's for a hangover on a basis of the intoxication degree and the history of the health condition.

The above arrangement allows the tendency of the individual user's for a hangover to be determined, and is thereby likely to allow the user to understand the tendency of the user's physical condition after drinking.

An intoxication degree determining system as a preferable embodiment of the present invention is arranged such that the computation section determines additionally on a basis of the tendency for a hangover whether the user is able to continue drinking.

The above arrangement allows the user to be advised to drink moderately on the additional basis of the user's tendency for a hangover, and is thereby likely to further increase the level of satisfaction on drinking occasions.

An intoxication degree determining system as a preferable embodiment of the present invention further includes: a personal information storing section configured to store information on at least one of (i) a hereditary trait of the user's associated with fitness of the user's to drink and (ii) a drinking habit of the user's, wherein the computation section determines additionally on a basis of at least one of the hereditary trait and the drinking habit whether the user is able to continue drinking.

The above arrangement allows the user to be advised to drink moderately on the additional basis of at least one of the user's fitness to drink and the user's drinking habit, and is thereby likely to allow the user to further enjoy drinking.

An intoxication degree determining system as a preferable embodiment of the present invention further includes: a user managing section configured to identify each of a plurality of users, wherein the intoxication degree determining system is configured to determine an intoxication degree of each user's.

The above arrangement allows the intoxication state of each of a plurality of users to be determined without requiring prior learning or accurate measurement.

An intoxication degree determining system as a preferable embodiment of the present invention is arranged such that the user managing section is further configured to identify a participant, the participant being a user participating in a particular drinking occasion, and the computation section is configured to evaluate a level of satisfaction on the particular drinking occasion on a basis of an intoxication degree of the participant's.

The above arrangement is likely to allow drinking occasions to be satisfying for many participants.

An intoxication degree determining system as a preferable embodiment of the present invention is arranged such that the output section and the input section are an output section and an input section of a portable information terminal.

The above arrangement allows the output section and the input section to be used by means of portable information terminals that many users are accustomed to using, and is likely to allow the user to easily understand how to use the system of the present invention.

Additional features and advantages of the present invention will be made clearer by the description of the exemplary and non-limiting embodiments below, which are described with reference to the drawings.

### Brief Description of Drawings

Fig. 1 is a diagram illustrating the configuration of an intoxication degree determining system according to Embodiment 1 of the present invention.
Fig. 2 is a determination flowchart according to Embodiment 1 of the present invention.
Fig. 3 is a diagram schematically illustrating an intoxication degree determining system according to Embodiment 2 of the present invention.

### Description of Embodiments

### [Embodiment 1]

With reference to drawings, the description below deals with Embodiment 1 of an intoxication degree determining system, an intoxication degree determining method, and an intoxication degree determining program according to the present invention. The example described below is an intoxication degree determining method involving use of an intoxication degree determining system 100 including a smartphone 10 and a server device 20. The smartphone 10 and the server device 20 each include an intoxication degree determining program according to the present invention installed thereon.

### [Basic Configuration of Intoxication Degree Determining System]

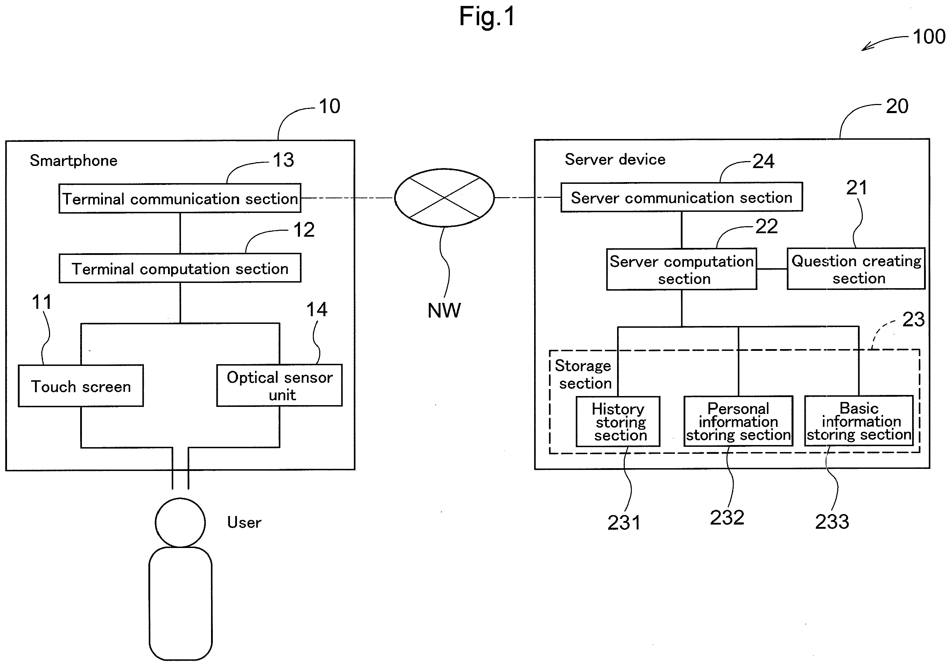

As illustrated in Fig. 1, the intoxication degree determining system 100 includes a smartphone 10 and a server device 20. The smartphone 10 (which is an example of the "portable information terminal") includes a touch screen 11 (which is an example of the "output section" and the "input section"), a terminal computation section 12, a terminal communication section 13, and an optical sensor unit 14 (which is an example of the "sensing section"). The server device 20 includes a question creating section 21, a server computation section 22 (which is an example of the "computation section"), a storage section 23, and a server communication section 24. The storage section 23 includes a history storing section 231, a personal information storing section 232, and a basic information storing section 233. The smartphone 10 and the server device 20 are communicable with each other over a network NW with use of the respective communication functions of the terminal communication section 13 and the server communication section 24. The terminal computation section 12 controls the respective operations of the other sections of the smartphone 10, whereas the server computation section 22 controls the respective operations of the other sections of the server device 20.

The question creating section 21 is configured to create questions for a drinking smartphone user to answer. The server device 20 transmits a question(s) selected by the question creating section 21 to the smartphone 10. The smartphone 10 then presents the question to the user of the smartphone 10 by displaying the question on the touch screen 11. The user can input an answer to the question into the smartphone 10 by touching the touch screen 11.

The optical sensor unit 14 is present at a position that the user's finger or thumb touches when the user naturally holds the smartphone 10. This allows the optical sensor unit 14 to measure the color of the user's finger or thumb simultaneously when the user operates the smartphone 10 to answer the question. The optical sensor unit 14, for this purpose, functions like a publicly known sensor for sensing a pulse wave on the basis of reflected light, and is capable of measuring the user's pulse wave and blood pressure.

The smartphone 10 transmits to the server device 20 the answer inputted by the user on the touch screen 11 and data on the user's pulse and blood pressure measured by the optical sensor unit 14. The server computation section 22 then determines the user's intoxication degree on the basis of the answer and the pulse and blood pressure.

The basic information storing section 233 of the storage section 23 stores information on a list of questions to be presented to the user, the correlation between answers and the intoxication degree, and the correlation between pulses and blood pressures and the intoxication degree. The history storing section 231 of the storage section 23 stores information on the intoxication degree associated with the user's past drinking and how the intoxication state changed over time, and information on whether the user suffered hangover the following morning. The storage section 23 accumulates these information items on the basis of a history of determination of the intoxication degree and intoxication state during drinking, and follow-up surveys conducted on the user. The personal information storing section 232 of the storage section 23 stores information on the user's hereditary traits associated with the user's fitness to drink and the user's drinking habit. The storage section 23 accumulates these information items on the basis of surveys on the user.

### [Intoxication Degree Determining Function]

The description below deals with various functions of the intoxication degree determining system 100 according to the present embodiment. First, the intoxication degree determining system 100 has an intoxication degree determining function (intoxication degree determining method). As described above, the question creating section 21 is configured to create questions for a drinking smartphone user to answer. Specifically, the question creating section 21 selects a single question from a list of questions stored in advance in the basic information storing section 233. The questions created are more difficult to answer correctly as the user's intoxication degree is higher. In terms of their level, the questions include such questions as "Can you touch the hand with which you will lose a rock paper scissors game against the hand displayed on the screen?" and "Can you touch only 'Nu's (a Japanese character) among all the 'Mu's (another Japanese character) and 'N's displayed on the screen?". The intoxication degree determining system 100 assumes a person who answers the question to have a cognitive level of the average adult, in view of the object of the present invention of determining the intoxication degree.

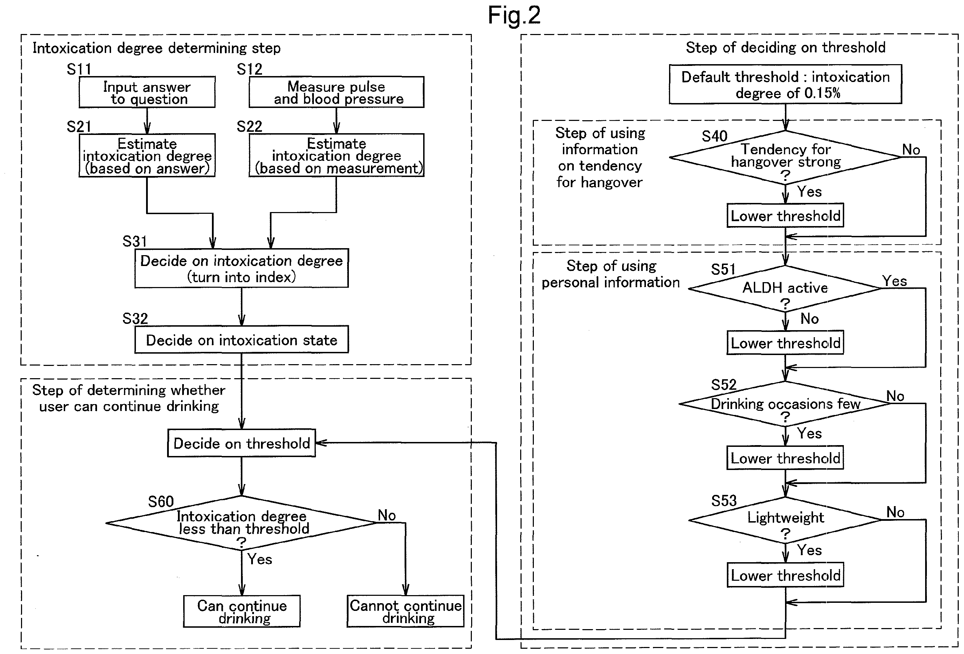

When the user has activated the intoxication degree determining program on the smartphone 10, the smartphone 10 requests the server device 20 to create a question(s). In response to the request, the question creating section 21 creates a question(s). The server device 20 transmits the question selected by the question creating section 21 to the smartphone 10. The smartphone 10 then presents the question to the user of the smartphone 10 by displaying the question on the touch screen 11. The user inputs an answer to the question into the smartphone 10 by touching the touch screen 11 (see Fig. 2, step S11). The touch screen 11 displays content corresponding to each question and a possible answer. If the question is, for example, the above question "Can you touch the hand with which you will lose a rock paper scissors game against the hand displayed on the screen?", the touch screen 11 displays images of a hand for the rock paper scissors game for the question and three hand options for the user to choose from as an answer.

Further, when the user inputs an answer to the question on the touch screen 11, the optical sensor unit 14 simultaneously measures the user's pulse and blood pressure (see Fig. 2, step S12).

When the user has inputted an answer to a question on the touch screen 11, the smartphone 10 transmits to the server device 20 the answer and information on the time length required by the user to answer the question. The server computation section 22 determines whether the answer received is correct, and stores in the history storing section 231 the answer, information on whether the answer is correct, and information on the time length required. During this operation, the smartphone 10 also transmits to the server device 20 information on the user's pulse and blood pressure measured simultaneously with the user's answer input. The server computation section 22 stores this information in the history storing section 231 as well.

The server computation section 22 estimates the user's intoxication degree at the time of answering the question by checking each answer, whether the answer is correct, and the required time length against the correlation (information on which is stored in advance in the basic information storing section 233) between (i) answers, whether the answers are correct, and required time lengths and (ii) the intoxication degree (see Fig. 2, step S21). The wrong answer rate and the time length required to answer a question, in particular, are positively correlated with the intoxication degree because they increase as a result of alcoholic intoxication affecting the user's brain.

During the above operation, the server computation section 22 simultaneously estimates the user's intoxication degree at the time of answering the question by checking the measured pulse and blood pressure of the user's against the correlation (information on which is stored in advance in the basic information storing section 233) between (i) pulses and blood pressures and (ii) the intoxication degree (see Fig. 2, step S22). A person's pulse and blood pressure are known to be positively correlated with the person's intoxication degree.

The server computation section 22 then converts the user's intoxication degree at the time of answering the question into an index (expressed in a percentage) comprehensively on the basis of the intoxication degree based on the user's answer and the intoxication degree based on the measurement (see Fig. 2, step S31). Next, the server computation section 22 classifies the user's intoxication state into one of the following levels: the pleasant period, the slightly intoxicated period, the early intoxicated period, the intoxicated period, the severely intoxicated period, and the deep sleep period (see Fig. 2, step S32). The server computation section 22 stores in the history storing section 231 information on the index of the intoxication degree and the intoxication state determined, together with a time stamp of each answer input.

### [Function of Determining Whether User Can Continue Drinking]

The intoxication degree determining system 100 according to the present embodiment also has the function of determining whether the user is able to continue drinking. The server computation section 22 performs this function by determining whether the user can continue drinking, on the basis of the index of the intoxication degree and the intoxication state determined (see Fig. 2, step S60). Specifically, the server computation section 22 determines that the user can continue drinking if the intoxication degree is less than 0.15% (that is, if the intoxication state is the pleasant period, the slightly intoxicated period, or the early intoxicated period) and determines that the user cannot continue drinking if the intoxication degree is not less than 0.15% (that is, if the intoxication state is the intoxicated period, the severely intoxicated period, or the deep sleep period). The server computation section 22 stores in the history storing section 231 information on whether the user can continue drinking.

The server device 20 transmits to the smartphone 10 information on the intoxication degree, the intoxication state, and whether the user can continue drinking, determined as above. The smartphone 10 then presents the information to the user of the smartphone 10 by displaying the information on the touch screen 11.

### [Hangover Tendency Determining Function]

The description below deals with the hangover tendency determining function of the intoxication degree determining system 100 according to the present embodiment. The question creating section 21 creates a question(s) about the user's health condition eight hours after the end of the drinking occasion for which the intoxication degree was determined as above (which is an example of the "follow-up time point, the follow-up time point being a predetermined time point after the user ended drinking"). The questions created are intended to ask whether the user has typical symptoms of a hangover, specifically such questions as "Did you get up this morning as you scheduled?", "Do you have a headache?", and "Are you feeling dizzy?".

The server device 20 transmits the question selected by the question creating section 21 to the smartphone 10. The smartphone 10 then presents the question to the user of the smartphone 10 by displaying the question on the touch screen 11. The user inputs an answer to the question into the smartphone 10 by touching the touch screen 11. The smartphone 10 transmits the answer inputted by the user to the server device 20. The server computation section 22 then converts the user's hangover state into an index on the basis of the answer received. The server computation section 22 stores in the history storing section 231 the answer and the index of the hangover state in association with the intoxication degree and intoxication state determined on the drinking occasion immediately before the user inputted the answer.

The history storing section 231 thus accumulates information on (i) a history of the intoxication degree and intoxication state during drinking and (ii) the hangover state on the day following the drinking occasion. This accumulated information allows the server computation section 22 to determine the user's tendency for a hangover (that is, the correlation between drinking and the probability of a hangover). The server computation section 22 calculates the probability of a hangover on subsequent drinking occasions on the basis of the intoxication degree and the tendency for a hangover. The server device 20 transmits information on the calculated probability of a hangover to the smartphone 10. The smartphone 10 displays the information on the touch screen 11.

The user's tendency for a hangover can serve as an additional ground for determination when the server computation section 22 determines whether the user can continue drinking. Specifically, for a user who has a strong tendency for a hangover (that is, who highly probably suffers a hangover), the server computation section 22 lowers the threshold of the intoxication degree for determining that the user cannot continue drinking (see Fig. 2, step S40).

### [Personal Information Using Function]

The description below deals with the personal information using function of the intoxication degree determining system 100 according to the present embodiment. The personal information storing section 232 of the storage section 23 stores personal information on the user's drinking. Personal information on a user's drinking includes information on the user's hereditary traits, drinking habit, and body. The storage section 23 accumulates these information items on the basis of surveys on the user. Specifically, the above information items are, for example, inputted by a user himself/herself before the user uses the intoxication degree determining function.

The hereditary traits of a user associated with the user's fitness to drink can be determined with use of an alcohol patch test, for example. On the basis of the result of the alcohol patch test conducted, the user selects on the touch screen 11 whether the user's genotype is ALDH2 (aldehyde dehydrogenase 2) active, ALDH2 less-active, or ALDH2 inactive. The smartphone 10 transmits the genotype information inputted by the user to the server device 20. The server device 20 then stores the genotype information in the personal information storing section 232.

Information on a user's drinking habit includes such information as the number of drinking occasions per week, the amount of alcohol consumption on a drinking occasion, and favorite alcohol drinks and brands. Information on a user's body includes such information as the user's height, weight, sex, and age. The user inputs these information items on the touch screen 11 as well. The smartphone 10 transmits the drinking habit information inputted by the user to the server device 20. The server device 20 then stores the drinking habit information in the personal information storing section 232.

The information on the user's hereditary traits, drinking habit, and body can serve as an additional ground for determination of the user's tendency for a hangover when the server computation section 22 determines whether the user can continue drinking. Specifically, for a user who meets such a condition(s) as having an ALDH2 less-active or inactive genotype, usually having only a few drinking occasions, and/or being lightweight, the server computation section 22 lowers the threshold of the intoxication degree for determining that the user cannot continue drinking, as compared to a user who does not meet such a condition(s) (see Fig. 2, step S51).

### [Embodiment 2]

The description below deals with an intoxication degree determining system 200 according to Embodiment 2 of the present invention with reference to a drawing. The description below does not deal with matters already described above for Embodiment 1.

### [Basic Configuration of Intoxication Degree Determining System]

Embodiment 2 includes a server device 20' and a plurality of smartphones 10a to 10x, the former and the latter being communicable with each other over a network NW The smartphones 10a to 10x each have a configuration identical to that of the smartphone 10 for Embodiment 1. The respective users Pa to Px of the smartphones 10a to 10x can thus each independently use the above-described functions of the intoxication degree determining system 100 according to Embodiment 1 by means of the corresponding one of the smartphones 10a to 10x and the server device 20'.

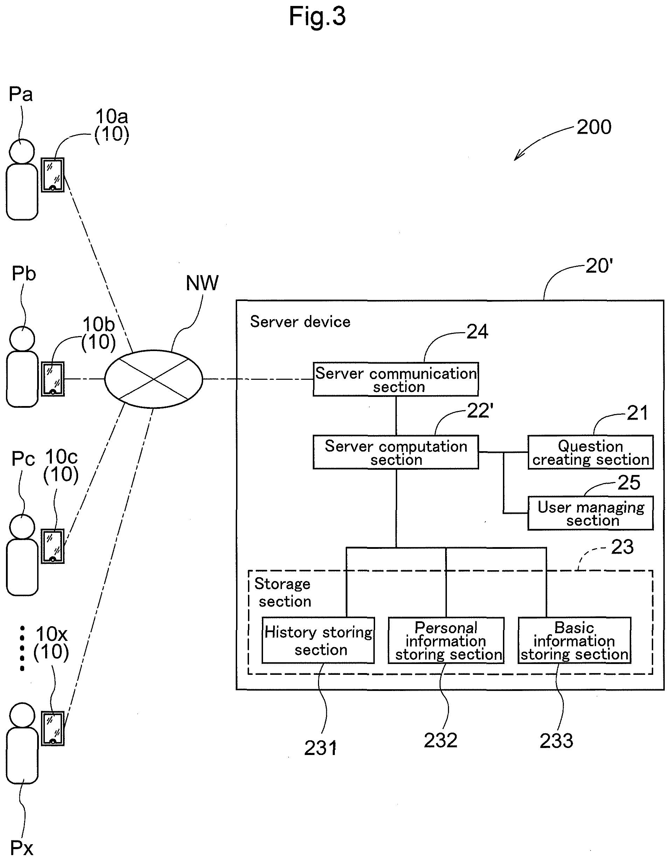

The server device 20' according to Embodiment 2 includes elements similar to those of the server device 20 according to Embodiment 1, and additionally includes a user managing section 25 (see Fig. 3). The user managing section 25 identifies and manages the individual users Pa to Px in association with login IDs assigned respectively to the users. Specifically, the user managing section 25 controls the server computation section 22' so that the functions described for Embodiment 1 are performed for each of the individual users Pa to Px with use of various information items about that particular user. The information items are specifically information on the answer(s) to a question(s), whether the answer is correct, and the time length required to answer the question; the pulse and blood pressure measured; a history of the intoxication degree and intoxication state; a history of the hangover state and the tendency for a hangover; and the hereditary traits, drinking habit, and body (personal information).

### [Drinking Occasion Evaluating Function]

The server device 20' according to Embodiment 2 is capable of performing, not only the functions described for Embodiment 1, but also a function of evaluating the level of satisfaction on a drinking occasion on the basis of, for example, the intoxication degree of each of a plurality of users present together on the drinking occasion.

The user managing section 25 identifies each user (participant) present on a drinking occasion as an evaluation target on the basis of the login ID. The server computation section 22' creates an index indicative of the level of satisfaction on the drinking occasion on the basis of each participant's intoxication degree and intoxication state determined during the drinking occasion. Specifically, the server computation section 22' increases the index if many participants are in the pleasant period or the slightly intoxicated period in terms of the intoxication state, and decreases the index if any participant is in the severely intoxicated period or the deep sleep period in terms of the intoxication state.

If the server computation section 22' has determined on the basis of the above-described function of determining whether the user can continue drinking that a participant cannot continue drinking, not only the smartphone 10 of that participant but also the respective smartphones 10 of the other participants display information to the effect that the participant cannot continue drinking.

### [Advantages of Embodiments]

The above-described intoxication degree determining system 100 according to Embodiment 1 of the present invention is capable of determining the user's intoxication degree during a drinking occasion on the basis of objective indicators, and also capable of determining, on the basis of the intoxication degree determined, whether the user can continue drinking. Further, the intoxication degree determining system 100 is capable of determining whether the user can continue drinking, on the basis of each user's tendency for a hangover and personal information. The intoxication degree determining system 100 is thus capable of suggesting to each user an amount of drinking that likely allows the user to feel good, such as an amount of drinking that allows the user to be in the pleasant period or the slightly intoxicated period. These functions allow drinking occasions to be enjoyable.

The intoxication degree determining system 200 according to Embodiment 2 of the present invention increases the index when many participants are drinking moderately, thereby allowing each participant to be advised to drink moderately. The intoxication degree determining system 200 also allows a participant to advise another participant to stop drinking if the other participant should not continue drinking any more. These features likely increase the level of satisfaction on a drinking occasion.

### [Other Embodiments]

Finally, the description below deals with intoxication degree determining systems, intoxication degree determining methods, and intoxication degree determining programs as other embodiments of the present invention. The arrangement disclosed for any embodiment below is combinable with the arrangement disclosed for any other embodiment unless such a combination causes any inconvenience.

Embodiment 1 described above is an example in which the intoxication degree determining system 100 is capable of performing an intoxication degree determining function, a function of determining whether the user can continue drinking, a hangover tendency determining function, and a personal information using function. The present invention is, however, not limited to such an arrangement. The intoxication degree determining system of the present invention at least includes a question creating section configured to create a question for a drinking user to answer; an output section configured to present the user with the question; an input section configured to receive an operation by the user of inputting an answer to the question; and a computation section configured to determine an intoxication degree of the user's on the basis of the answer. Regarding the other three functions (namely, the function of determining whether the user can continue drinking, the hangover tendency determining function, and the personal information using function), the intoxication degree determining system of the present invention may be capable of performing one, two, all, or even none of them.

Embodiment 1 described above is an example in which the intoxication degree determining system 100 includes a smartphone 10 and a server device 20, the smartphone 10 including a touch screen 11, a terminal computation section 12, a terminal communication section 13, and an optical sensor unit 14, the server device 20 including a question creating section 21, a server computation section 22, a storage section 23, and a server communication section 24. The present invention is, however, not limited to such an arrangement. The intoxication degree determining system of the present invention may alternatively include a single device that serves as each of the question creating section, the output section, the input section, and the computation section.

Embodiment 1 described above is an example in which the server computation section 22 estimates a user's intoxication degree on the basis of the user's answer(s) to a question(s), whether the answer is correct, and the time length required by the user to answer the question, the question being created by the question creating section 21 for the intoxication degree determining function and being more difficult to answer correctly as the user's intoxication degree is higher (such as "Can you touch the hand with which you will lose a rock paper scissors game against the hand displayed on the screen?"). For the intoxication degree determining function, however, the question creating section may alternatively create a question(s) about the kind(s) and amount(s) of the beverage(s) that the user has consumed by the time the user answers the question, so that the computation section estimates the user's intoxication degree on the basis of the user's answer to the question. With this embodiment, the intoxication degree determining function allows the computation section to estimate the order and respective amounts of alcoholic beverages that each user has consumed, on the basis of the kind(s) and amount(s) of the beverage(s) that the user has consumed. In this case, the computation section is capable of estimating the user's intoxication degree on the basis of the correlation between (i) the order and respective amounts of the alcoholic beverages and (ii) the intoxication degree. Further, the computation section may also use, as a ground for the intoxication degree estimation, such information as each user's physical predisposition (for example, whether for each kind of alcoholic beverage, the user has a high tolerance or not). This embodiment also allows each user to check a history of what beverages the user has consumed (specifically, such information as the kinds, amounts, order, and times). The computation section may use any one of the above methods for the intoxication degree estimation, combine two or more of the above methods, or combine one or more of the above methods with a different estimation method(s).

Embodiment 1 described above is an example in which the smartphone 10 displays on the touch screen 11 information on whether the user can continue drinking. The output section may alternatively output such information together with additional information based thereon. The output section may output, for example, a suggestion of an alcoholic beverage that the user should drink next if the user can continue drinking and a suggestion of a non-alcoholic beverage that the user should drink next if the user cannot continue drinking. The output section may further alternatively display an image of a discount coupon together with the suggestion. The storage section may be capable of storing information on whether the user adopted the suggestion.

Embodiment 1 described above is an example in which the intoxication degree determining system 100 includes a smartphone 10 including an optical sensor unit 14 capable of functioning as a sensor for sensing a pulse wave on the basis of reflected light and converts the user's intoxication degree at the time of answering the question into an index comprehensively on the basis of the intoxication degree based on the user's answer and the intoxication degree based on the measurement. The present invention is, however, not limited to such an arrangement. The intoxication degree determining system of the present invention may omit such a measurement section and determine the intoxication degree on the basis of the answer only.

Embodiment 1 described above is an example in which the intoxication degree determining system 100 converts the user's intoxication degree into an index expressed in a percentage and classifies the user's intoxication state into one of the following levels: the pleasant period, the slightly intoxicated period, the early intoxicated period, the intoxicated period, the severely intoxicated period, and the deep sleep period. The present invention is, however, not limited to such an arrangement. The intoxication degree determining system of the present invention may alternatively determine the user's intoxication degree by any method such as by converting the user's intoxication degree into a numerical point, a graph, or an icon. Further, the intoxication degree determining system of the present invention may classify the user's intoxication state into one of fewer or more than the above six levels.

Embodiment 1 described above is an example in which the intoxication degree determining system 100 includes its output section and input section in the integrated form of the touch screen 11 of the smartphone 10. The present invention is, however, not limited to such an arrangement. The intoxication degree determining system of the present invention may alternatively include separate output and input devices as its output section and input section. Further alternatively, the output section and input section may be of such a portable information terminal as a smartwatch, smartglasses, virtual reality goggles, a tablet computer, a laptop personal computer, or a portable telephone.

Embodiment 1 described above is an example in which the intoxication degree determining system 100 includes a smartphone 10 including an optical sensor unit 14 capable of measuring the user's pulse and blood pressure. The present invention is, however, not limited to such an arrangement. In the case where the intoxication degree determining system of the present invention includes a sensor section, the sensor section may be provided for a device that is integrated with one of the question creating section, the output section, the input section, and the computation section or that is separate from any of these sections. The intoxication degree determining system of the present invention may, for instance, include (i) as a variation of the smartphone 10 for Embodiments 1 and 2, a smartphone including a question creating section, an output section, an input section, and a computation section and (ii) a casing attachable to the smartphone, the casing being provided with a sensor section capable of sensing at least one of the user's pulse, blood pressure, and blood alcohol concentration and a communication section capable of communicating with the smartphone.

Embodiment 1 described above is an example in which the intoxication degree determining system 100 includes a smartphone 10 including an optical sensor unit 14 capable of measuring the user's pulse and blood pressure. The present invention is, however, not limited to such an arrangement. In the case where the intoxication degree determining system of the present invention includes a sensor section, the sensor section may be capable of sensing at least one of the user's pulse rate, blood pressure, and blood alcohol concentration.

Embodiment 1 described above is an example in which the server computation section 22 determines that the user can continue drinking if the intoxication degree is less than 0.15%, in the case where the server computation section 22 does not use information on the user's tendency for a hangover or personal information. The present invention is, however, not limited to such an arrangement. The threshold of the intoxication degree for determining that the user cannot continue drinking is changeable, and may be set on the basis of, for example, the office regulations that the user need to obey, guidance given by the user's attending doctor, and/or the user's preferences. The threshold may be changed by the user freely or by only someone authorized to change the threshold (such as the user's attending doctor).

Embodiment 1 described above is an example in which for a user who has a strong tendency for a hangover, the server computation section 22 lowers the threshold of the intoxication degree for determining that the user cannot continue drinking. For a user who has a weak tendency for a hangover, the computation section may raise the threshold.

Embodiment 1 described above is an example in which for a user who meets such a condition(s) as having an ALDH2 less-active or inactive genotype, usually having only a few drinking occasions, and/or being lightweight, the server computation section 22 lowers the threshold of the intoxication degree for determining that the user cannot continue drinking. For a user who does not meet the condition, the computation section may raise the threshold.

Embodiment 1 described above is an example in which the smartphone 10 displays a question(s) for the user about the user's health condition eight hours after the end of the last drinking occasion for which the intoxication degree was determined, and the server computation section 22 converts the user's hangover state into an index on the basis of the answer to the question, that is, an example in which the follow-up time point for the present invention is eight hours after the end of the last drinking occasion. The present invention is, however, not limited to such an arrangement. The follow-up time point for the present invention may be any time point within a time period in which the user may have a hangover symptom after the end of the last drinking occasion. Examples of the follow-up time point include a time point that is a predetermined time period after the end of the last drinking occasion and a predetermined time point on the day following the last drinking occasion.

Embodiment 2 described above is an example in which the intoxication degree determining system 200 manages individual users on the basis of the login ID of each user. The present invention is, however, not limited to such an arrangement. With two or more users involved, the intoxication degree determining system of the present invention may alternatively identify and manage individual users by another publicly known method such as fingerprint authentication, voiceprint authentication, or facial recognition.

Embodiment 2 described above is an example in which the intoxication degree determining system 200 creates an index indicative of the level of satisfaction on a drinking occasion on the basis of each participant's intoxication degree. The present invention is, however, not limited to such an arrangement. The intoxication degree determining system of the present invention may alternatively determine the level of satisfaction on a drinking occasion on the basis of another determination criterion. The intoxication degree determining system may, for instance, determine if many participants have a pulse and blood pressure each within a predetermined range of desirable values that the level of satisfaction is high on the drinking occasion, where many participants are feeling relaxed.

It should be understood that with respect to other arrangements as well, the embodiments disclosed herein are illustrative in all respects and do not limit the scope of the present invention. Persons skilled in the art will readily understand that the present invention can be modified as appropriate without departing from the scope of the present invention. The present invention thus naturally covers in its scope any embodiment as modified without departing from the scope of the present invention.

### Industrial Applicability

The present invention is applicable to, for example, a smartphone capable of determining the user's intoxication degree.

### Reference Signs List

- 100: Intoxication degree determining system according to Embodiment 1
- 200: Intoxication degree determining system according to Embodiment 2
- 10: Smartphone
- 11: Touch screen
- 12: Terminal computation section
- 13: Terminal communication section
- 14: Optical sensor unit
- 20: Server device
- 21: Question creating section
- 22: Server computation section
- 23: Storage section
- 231: History storing section
- 232: Personal information storing section
- 233: Basic information storing section
- 24: Server communication section
- 25: User managing section

## Claims

1. An intoxication degree determining system, comprising:
a question creating section configured to create a question for a drinking user to answer;
an output section configured to present the user with the question;
an input section configured to receive an operation by the user of inputting an answer to the question; and
a computation section configured to determine an intoxication degree of the user's on a basis of the answer.

2. The intoxication degree determining system according to claim 1, further comprising:
a sensor section configured to sense at least one of a pulse rate, a blood pressure, and a blood alcohol concentration of the user's, wherein
the computation section determines the intoxication degree of the user's additionally on a basis of the at least one of the pulse rate, the blood pressure, and the blood alcohol concentration of the user's.

3. The intoxication degree determining system according to claim 1 or 2, wherein
the computation section determines on a basis of the intoxication degree whether the user is able to continue drinking, and
the output section is configured to present the use with a result of the determination of whether the user is able to continue drinking.

4. The intoxication degree determining system according to claim 3, further comprising:
a history storing section configured to store a history of the intoxication degree of the user's, wherein
the question creating section is configured to create a follow-up question, the follow-up question being a question for the user to answer about a health condition of the user's at a follow-up time point, the follow-up time point being a predetermined time point after the user ended drinking,
the history storing section is configured to further store a history of the health condition, which is based on an answer by the user to the follow-up question, and
the computation section is configured to determine a tendency of the user's for a hangover on a basis of the intoxication degree and the history of the health condition.

5. The intoxication degree determining system according to claim 4, wherein
the computation section determines additionally on a basis of the tendency for a hangover whether the user is able to continue drinking.

6. The intoxication degree determining system according to any one of claims 3 to 5, further comprising:
a personal information storing section configured to store information on at least one of (i) a hereditary trait of the user's associated with fitness of the user's to drink and (ii) a drinking habit of the user's, wherein
the computation section determines additionally on a basis of at least one of the hereditary trait and the drinking habit whether the user is able to continue drinking.

7. The intoxication degree determining system according to any one of claims 1 to 6, further comprising:
a user managing section configured to identify each of a plurality of users, wherein
the intoxication degree determining system is configured to determine an intoxication degree of each user's.

8. The intoxication degree determining system according to claim 7, wherein
the user managing section is further configured to identify a participant, the participant being a user participating in a particular drinking occasion, and
the computation section is configured to evaluate a level of satisfaction on the particular drinking occasion on a basis of an intoxication degree of the participant's.

9. The intoxication degree determining system according to any one of claims 1 to 8, wherein
the output section and the input section are an output section and an input section of a portable information terminal.

10. An intoxication degree determining method, comprising:
creating a question for a drinking user to answer;
presenting the user with the question;
receiving an answer to the question from the user; and
determining an intoxication degree of the user's on a basis of the answer.

11. An intoxication degree determining program configured to cause a computer to:
create a question for a drinking user to answer;
present the user with the question;
receive an input of an answer to the question from the user; and
determine an intoxication degree of the user's on a basis of the answer.
